## Europäisches Patentamt
## European Patent Office
## Office européen des brevets

(19)

(11) Veröffentlichungsnummer : **0 040 698**
**B1**

(12) **EUROPÄISCHE PATENTSCHRIFT**

(45) Veröffentlichungstag der Patentschrift :
28.12.83

(21) Anmeldenummer : 81103114.5

(22) Anmeldetag : 24.04.81

(51) Int. Cl.³ : **C 07 D213/02, C 07 D213/10,**
**C 07 D213/12**

(54) Verfahren zur Herstellung von 3-Picolin.

(30) Priorität : 23.05.80 CH 4052/80

(43) Veröffentlichungstag der Anmeldung :
02.12.81 Patentblatt 81/48

(45) Bekanntmachung des Hinweises auf die Patenterteilung : 28.12.83 Patentblatt 83/52

(84) Benannte Vertragsstaaten :
AT BE CH DE FR GB IT LI LU NL SE

(56) Entgegenhaltungen :
DE-A- 1 903 878
DE-A- 2 337 087
GB-A-   971 174
GB-A- 1 182 705
GB-A- 1 208 569
Chemical Economy & Engineering Review, June
1975, no 6 Seite 34,35
Chem. Techn, 12, Jg Heft 12, December 1970, Seite
745-748
Die Akte enthält technische Angaben, die nach dem
Eingang der Anmeldung eingereicht wurden und die
nicht in dieser Patentschrift enthalten sind.

(73) Patentinhaber : **LONZA AG**
**Gampel/Wallis (CH)**

(72) Erfinder : **Dinkel, Rolf, Dr.**
**Sandstrasse 5**
**VISP (Kanton Wallis) (CH)**

(74) Vertreter : **von Füner, Alexander, Dr. et al**
**Patentanwälte v. Füner, Ebbinghaus, Finck Mariahilf-**
**platz 2 & 3**
**D-8000 München 90 (DE)**

## Verfahren zur Herstellung von 3-Picolin

Die vorliegende Erfindung betrifft ein Verfahren zur Herstellung von 3-Picolin.

Pyridinbasen stellen wichtige Zwischenprodukte in der chemischen Industrie dar, so z. B. bei der Herstellung von Nicotinsäure oder Nicotinsäureamid. Es sind verschiedene Verfahren zur Herstellung von Pyridinbasen bekannt.

2-Methyl-5-Aethylpyridin wird heute großtechnisch im Flüssigphasenverfahren aus Acetaldehyd oder Paraldehyd und Ammoniak in Gegenwart verschiedenster Katalysatoren, wie z. B. Ammoniumsalze, hergestellt. Als Nebenprodukt fallen kleine Mengen an 2- und 4-Picolin an.

2- und 4-Picolin werden heute in Gasphasenreaktion bei Temperaturen von ca. 400 °C aus Acetaldehyd und Ammoniak unter Verwendung von Festbett- oder Fließbettkatalysatoren auf Basis Aluminiumsilikat hergestellt (siehe Chemical Economy & Engineering Review, June 1975, No. 6, Seite 34 und 35).

Für die Erzeugung von Pyridin sowie von 3-Picolin, welches immer größere Bedeutung bekommt, werden heute Gasphasenreaktionen angewendet, wobei durch Zugabe von Formaldehyd zum Acetaldehyd die Bildung von 2- und 4-Picolin zu Gunsten von 3-Picolin unterdrückt wird. Auch diese Umsetzungen finden im Festbett oder Fließbett mit Aluminiumsilikat als Katalysator bei Temperaturen von etwa 400 °C statt. Nach diesen Verfahren werden Ausbeuten an 3-Picolin in der Größenordnung von höchstens 40 bis 44 % erzielt. Daneben fallen große Mengen an Pyridin an.

Es ist auch bekannt, daß anstelle von gesättigten Aldehyden von ungesättigten Aldehyden, wie z. B. Acrolein oder Crotonaldehyd ausgegangen werden kann. Diese Reaktionen finden in der Gasphase bei hohen Temperaturen statt ; die Ausbeuten liegen im wesentlichen gleich hoch wie bei der Verwendung von gesättigten Aldehyden als Ausgangsmaterial (siehe Chem. Techn., 12. Jg., Heft 12, Dezember 1970, Seite 745 bis 748, Hans-Joachim Übel, Karl-Klaus Moll und Manfred Mühlstädt, Untersuchungen zur Gewinnung von 3-Methylpyridin).

Ziel der vorliegenden Erfindung ist es, 3-Picolin in hohen Ausbeuten herzustellen, wobei die Bildung von Pyridin möglichst unterdrückt werden soll.

Erfindungsgemäß wird dies dadurch erreicht, daß man Acetaldehyd bzw. dessen Polymere und/oder Crotonaldehyd im Gemisch mit Formaldehyd bzw. dessen Polymere in wäßriger Phase bei einem Molverhältnis von Acetaldehyd : Formaldehyd von 1 : 0,5 bis 1 : 1,2 und/oder Crotonaldehyd : Formaldehyd von 1 : 1,0 bis 1 : 2,4 bei Temperaturen von 180 bis 280 °C im geschlossenen Gefäß in Gegenwart von 0,5 bis 3 Mol Ammoniumsalze pro Mol Aldehyd umsetzt.

Unter Acetaldehyd im Sinne der Erfindung sind auch dessen Polymere, wie z. B. Paraldehyd, zu verstehen ; unter Formaldehyd auch dessen Polymere, wie z. B. Trioxan.

Werden miteinander nicht mischbare flüssige Ausgangsmaterialien, wie z. B. Paraldehyd zusammen mit wäßrigem Formaldehyd, verwendet, so ist es vorteilhaft, zur Homogenisierung kleine Mengen Homogenisierungsmittel, wie Alkohole, cyclische Aether, vorzugsweise jedoch vorgebildetes 3-Picolin, einzusetzen.

Nach dem Verfahren der Erfindung wird überraschenderweise 3-Picolin in Ausbeuten von ca. 60 bis 70 % erhalten und die Bildung von Pyridin fast vollständig unterdrückt (unter 1 %). Als Nebenprodukte fallen 3-Aethylpyridin sowie kleine Mengen an 2,5-Dimethylpyridin, 3,5-Dimethylpyridin und 2-Methyl-5-äthylpyridin an.

Das Verfahren der Erfindung wird also mit einem Molverhältnis Acetaldehyd zu Formaldehyd von 1 : 0,5 bis 1 : 1,2, vorzugsweise von 1 : 0,8 bis 1 : 1,0, durchgeführt.

Wird Crotonaldehyd anstelle von Acetaldehyd verwendet, so verschiebt sich das Molverhältnis Crotonaldehyd zu Formaldehyd entsprechend auf 1 : 1,0 bis 1 : 2,4.

Die Reaktionstemperaturen liegen demnach bei 180 bis 280 °C, zweckmäßig bei 205 bis 240 °C, vorzugsweise bei 225 bis 235 °C. Die Reaktion wird in wäßriger Phase unter einem Druck, der sich bei der Reaktion im geschlossenen Gefäß bei vorgegebener Temperatur einstellt, durchgeführt.

Es ist vorteilhaft, während der Reaktion den Reaktionsansatz zu rühren.

Als Ammoniumsalze kommen Ammoniumsalze wie Ammoniumacetat, Ammoniumfluorid, Ammoniumchlorid, Ammoniumbifluorid, Ammoniumborat, Ammoniumbenzoat, Ammoniummolybdat und Ammoniumsulfid in Betracht. Vorzugsweise werden Ammoniumphosphate angewendet. Diese Ammoniumsalze können auch in situ aus den entsprechenden Komponenten gebildet werden.

Eine Variante des Verfahrens der Erfindung besteht darin, daß neben dem Ammoniumsalz auch zusätzlich Ammoniak, als wäßrige Lösung oder gasförmig, eingesetzt wird.

Die Menge an Ammoniumsalzen, die als wäßrige Lösungen in Konzentrationen im Bereich von 1 bis 80 Gew.-% Ammoniumsalz eingesetzt werden, liegen bei 0,5 bis 3 Mol pro Mol Aldehyd, zweckmäßig bei 0,6 bis 1,0 Mol pro Mol Aldehyd. Vorzugsweise beträgt die Konzentration der Ammoniumsalze in wäßriger Lösung 2,9 bis 3,7 Mol/l.

Der Start-pH-Wert der wäßrigen Reaktionslösung liegt zweckmäßig zwischen 7 bis 9.

Die Zugabe des Aldehyds erfolgt zweckmäßig nach Maßgabe seines Verbrauches. So ist es beispielsweise günstig, beim Arbeiten in einem 2 l-Behälter und bei Einsatz von 350 ml Aldehyd diesen kontinuierlich während 29 bis 92 Minuten zuzusetzen. Bei anderen Bedingungen sind die entsprechenden Zugabezeiten zu wählen.

Am Ende der gewünschten Reaktionsperiode wird die Temperatur auf etwa Raumtemperatur erniedrigt und das 3-Picolin auf bekannte Weise aus der Reaktionsmischung gewonnen. Eine Methode besteht darin, daß man das organische Material aus der wäßrigen Reaktionsmischung mit einem organischen Lösungsmittel, z. B. Benzol, Toluol, Xylol, Methylenchlorid, Chloroform, Aether und dergleichen, extrahiert. Das organische Lösungsmittel wird dann abgedampft und man erhält 3-Picolin durch fraktionierte Destillation. Im Rahmen der Erfindung können auch beliebige andere Methoden zur Abtrennung und Gewinnung des Produkts angewendet werden.

Ein Vorteil des neuen erfindungsgemässen Verfahrens ist auch der, dass die nach der Extraktion der Reaktionsmischung mit einem organischen Lösungsmittel erhaltene wässrige Ammoniumsalzphase nach Wiederanreicherung mit Ammoniak in den Reaktor zurückgeführt werden kann. Die wässrige Salzphase ist zusammengesetzt aus der ursprünglich in der Salzlösung vorhandenen Wassermenge, dem nicht umgesetzten Ammoniumsalz, der Säure des an der Umsetzung teilnehmenden Ammoniumsalzes sowie einen Mol Wasser für jedes Mol bei der Umsetzung verbrauchten Aldehyds. Die wässrige Salzphase wird daher mit Hilfe eines beliebigen bekannten Verfahrens konzentriert, z. B. durch durch Verdampfen, um das infolge der Kondensationsreaktion gebildete Wasser zu entfernen. Eine Wiederanreicherung der Salzphase wird dann dadurch erreicht, dass man die wässrige Lösung mit flüssigem Ammoniak bei Umgebungstemperatur unter Bildung des Ammoniumsalzes umsetzt.

Obgleich die Erfindung als diskontinuierliches Verfahren beschrieben worden ist, kann das Verfahren auch im Rahmen der vorliegenden Erfindung kontinuierlich betrieben werden. Bei einer Ausführungsform eines kontinuierlichen Verfahrens werden die Reaktionsteilnehmer kontinuierlich in einen geeigneten Druckreaktor eingeführt, aus dem ein Teil der Reaktionsmischung kontinuierlich abgezogen wird. Die Reaktionsprodukte werden daraus abgetrennt und unveränderte Reaktionsteilnehmer werden dann wieder ergänzt und in das Reaktionsgefäss zurückgeführt.

Das kontinuierliche Verfahren kann in jedem Reaktor durchgeführt werden, der eine innige Vermischung der Reaktionsteilnehmer unter heftigem Rühren gestattet, z. B. in einem kontinuierlich gerührten Tankreaktor.

Beispiel 1

1 130 ml einer 3,37 molaren wässrigen Lösung von Diammoniumhydrogenphosphat (pH 8,3) werden in einem 2-Liter-Autoklaven auf 235 °C erhitzt und bei 1 500 U/min. gerührt. In diese Lösung wird innert 63 Minuten kontinuierlich ein Gemisch aus 114,1 g Acetaldehyd und 219,2 g einer 32,0 %igen wässrigen Formaldehyd-Lösung eingepumpt (Molverhältnis = 1 : 0,90). Dabei variiert der Reaktionsdruck zwischen 38 und 40 Bar. Nach beendeter Zugabe des Aldehydgemisches wird die Reaktionsmasse während 10 Minuten bei 235 °C weitergerührt und dann auf Raumtemperatur abgekühlt.

Schliesslich erfolgt eine Extraktion mit 3 × 100 ml Toluol sowie eine gaschromatographische Analyse der vereinigten Toluolextrakte, wobei sich folgende Produkte mit den auf eingesetzten Acetaldehyd (A) bzw. Formaldehyd (F) bezogenen Ausbeuten ergeben : Pyridin 0,9 % (A) ; 3-Picolin 68,0 % (F) ; 3-Ethylpyridin 15,0 % (A) ; 2,5-Lutidin 2,5 % (A) ; 3,5-Lutidin 1,4 % (F) ; 2-Methyl-5-ethylpyridin 0,6 % (A).

Sämtliche gaschromatographischen Analysen wurden unter Verwendung eines internen Standards sowie unter Berücksichtigung von Flächenkorrekturfaktoren durchgeführt.

Beispiel 2

1 130 ml einer 3,38 molaren wässrigen Lösung von Diammoniumhydrogenphosphat (pH 8,3) werden in einem 2-Liter-Autoklaven auf 230 °C erhitzt und bei 1 500 U/min. gerührt. In diese Lösung wird innert 60 Minuten kontinuierlich ein Gemisch aus 121,8 g Acetaldehyd und 208,2 g einer 32,0 %igen wässrigen Formaldehyd-Lösung eingepumpt (Molverhältnis = 1 : 0,80). Dabei variiert der Reaktionsdruck zwischen 33 und 35 Bar. Nach beendeter Zugabe des Aldehydgemisches wird die Reaktionsmasse während 10 Minuten bei 230 °C weitergerührt und dann auf Raumtemperatur abgekühlt. Schliesslich erfolgt eine Extraktion mit 3 × 100 ml Toluol sowie eine gaschromatographische Analyse der vereinigten Toluolextrakte, wobei sich folgende Produkte mit den auf eingesetzten Acetaldehyd (A) bzw. Formaldehyd (F) bezogenen Ausbeuten ergeben : Pyridin 0,8 % (A) ; 3-Picolin 62,5 % (F) ; 3-Ethylpyridin 22,6 % (A) ; 2,5-Lutidin 3,6 % (A) ; 3,5-Lutidin 0,9 % (F) ; 2-Methyl-5-ethylpyridin 1,9 % (A).

Beispiel 3

1 060 ml einer 3,37 molaren wässrigen Lösung von Diammoniumhydrogenphosphat (pH 8,3) werden in einem 2-Liter-Autoklaven auf 222 °C erhitzt und bei 1 200 U/min. gerührt. In diese Lösung wird innert 68 Minuten kontinuierlich ein Gemisch aus 108,4 g Paraldehyd, 222,4 g einer 33,2 %igen wässrigen Formaldehyd-Lösung (Molverhältnis = 1 : 3,00) und 73,1 g 3-Picolin (Homogenisierungsmittel) eingepumpt. Nach beendeter Zugabe des Aldehydgemisches wird die Reaktionsmasse während 10 Minuten bei 222 °C weitergerührt und dann auf Raumtemperatur abgekühlt. Schliesslich erfolgt eine Extraktion mit 3 × 100 ml Toluol sowie eine gaschromatographische Analyse der vereinigten Toluolextrakte, wobei sich folgende Produkte mit den auf eingesetzten Paraldehyd bezogenen Ausbeuten ergeben : Pyridin 0,8 % ; 3-Picolin 55,2 % (ohne Anteil für Homogenisierung) ; 3-Ethylpyridin 10,0 % ; 2,5-Lutidin 1,9 % ; 3,5-Lutidin 1,4 % ; 2-Methyl-5-ethylpyridin 1,9 %.

## Beispiel 4

In 1 140 ml einer 10,0 molaren wässrigen Ammoniumacetat-Lösung (pH 8,1) werden 25,0 g Ammoniak gasförmig eingeleitet und das Gemisch anschliessend in einem 2-Liter-Autoklaven auf 230 °C erhitzt und bei 1 500 U/min. gerührt. In dieses wird innert 58 Minuten kontinuierlich ein Gemisch aus 122,2 g Acetaldehyd und 208,2 g einer 32,0 %igen wässrigen Formaldehyd-Lösung eingepumpt (Molverhältnis = 1 : 0,80). Dabei variiert der Reaktionsdruck zwischen 27 und 29 Bar. Nach beendeter Zugabe des Aldehydgemisches wird die Reaktionsmasse während 10 Minuten bei 230 °C weitergerührt und dann auf Raumtemperatur abgekühlt. Schliesslich erfolgt eine gaschromatographische Analyse des homogenen Reaktionsgemisches, welche folgende Produkte mit den auf eingesetzten Acetaldehyd (A) bzw. Formaldehyd (F) bezogenen Ausbeute ergibt : Pyridin 0,9 % (A) ; 3-Picolin 44,8 % (F) ; 3-Ethylpyridin 19,1 % (A) ; 2,5-Lutidin 4,0 % (A) ; 3,5-Lutidin 0,4 % (F) ; 2-Methyl-5-ethylpyridin 1,7 % (A).

## Beispiel 5

1 140 ml einer 3,40 molaren wässrigen Ammoniumacetat-Lösung (pH 7,4) werden in einem 2-Liter-Autoklaven auf 230 °C und bei 1 500 U/min. gerührt. In diese Lösung wird innert 59 Minuten kontinuierlich ein Gemisch aus 122,2 g Acetaldehyd und 208,2 g einer 32,0 %igen wässrigen Formaldehyd-Lösung eingepumpt (Molverhältnis = 1 : 0,80). Dabei variiert der Reaktionsdruck zwischen 26 und 28 Bar. Nach beendeter Zugabe des Aldehydgemisches wird die Reaktionsmasse während 10 Minuten bei 230 °C weitergerührt, dann auf Raumtemperatur abgekühlt und mit gasförmigem Ammoniak ein pH von 8,1 eingestellt. Schliesslich erfolgt eine gaschromatographische Analyse des nunmehr homogenen Reaktionsgemisches, welche folgende Produkte mit den auf eingesetzten Acetaldehyd (A) bzw. Formaldehyd (F) bezogenen Ausbeuten ergibt : Pyridin 1,3 % (A) ; 3-Picolin 53,4 % (F) ; 3-Ethylpyridin 14,8 % (A) ; 2,5-Lutidin 4,1 % (A) ; 3,5-Lutidin 0,7 % (F) ; 2-Methyl-5-ethylpyridin 1,9 % (A).

## Beispiel 6

1 140 ml einer 3,40 molaren wässrigen Lösung von Diammoniumhydrogenphosphat (pH 8,35) werden in einem 2-Liter-Autoklaven auf 230 °C erhitzt und bei 1 500 UpM gerührt. In diese Lösung wird innert 74 Minuten kontinuierlich ein Gemisch aus 117,7 g Acetaldehyd, 64,0 g Trioxan und 30,0 g 3-Picolin (Homogenisierungsmittel) eingepumpt (kalkulatorisches Molverhältnis Acetaldehyd : Formaldehyd = 1 : 0,78). Dabei variiert der Reaktionsdruck zwischen 32 und 34 Bar. Nach beendeter Zugabe des Eduktgemisches wird die Reaktionsmasse während 10 Minuten bei 230 °C weitergerührt und dann auf Raumtemperatur abgekühlt. Schließlich erfolgt eine Extraktion mit 3 × 100 ml Methylenchlorid sowie eine gaschromatographische Analyse der vereinigten Methylenchloridextrakte, wobei sich folgende Produkte mit den je nach theoretischem Aldehydbedarf auf eingesetzten Acetaldehyd (A) oder auf eingesetztes Trioxan (F) bezogenen Ausbeuten ergeben : Pyridin 0,7 % (A) ; 3-Picolin 23,8 % (F) (ohne Anteil für Homogenisierung) ; 3-Ethylpyridin 35,1 % (A) ; 2,5-Lutidin 6,4 % (A) ; 3,5-Lutidin 0,2 % (F) ; 2-Methyl-5-ethylpyridin 23,3 % (A).

## Beispiel 7

1 140 ml einer 3,40 molaren wässrigen Lösung von Diammoniumhydrogenphosphat (pH 8,35) werden in einem 2-Liter-Autoklaven auf 230 °C erhitzt und bei 1 500 UpM gerührt. In diese Lösung wird innert 58 Minuten kontinuierlich ein Gemisch aus 117,7 g Paraldehyd, 64,0 g Trioxan, 130 g Wasser und 100 g Ethanol eingepumpt (kalkulatorisches Molverhältnis Acetaldehyd : Formaldehyd = 1 : 0,79). Dabei variiert der Reaktionsdruck zwischen 32 und 38 Bar. Nach beendeter Zugabe des Eduktgemisches wird die Reaktionsmasse während 10 Minuten bei 230 °C weitergerührt und dann auf Raumtemperatur abgekühlt. Schließlich erfolgt eine Extraktion mit 3 × 100 ml Methylenchlorid sowie eine gaschromatographische Analyse der vereinigten Methylenchloridextrakte, wobei sich folgende Produkte mit den je nach theoretischem Aldehydbedarf auf eingesetztes Paraldehyd (A) oder auf eingesetztes Trioxan (F) bezogenen Ausbeuten ergeben : Pyridin 0,6 % (A) ; 3-Picolin 19,1 % (F) ; 3-Ethylpyridin 36,0 % (A) ; 2,5-Lutidin 7,1 % (A) ; 3,5-Lutidin 0,2 % (F) ; 2-Methyl-5-ethylpyridin 23,9 % (A).

## Beispiel 8

1 140 ml einer 3,40 molaren wässrigen Lösung von Diammoniumhydrogenphosphat (pH 8,35) werden in einem 2-Liter-Autoklaven auf 230 °C erhitzt und bei 1 500 UpM gerührt. In diese Lösung wird innerhalb 60 Minuten kontinuierlich ein Gemisch aus 64,4 g Crotonaldehyd, 39,2 g Acetaldehyd und 213,3 g einer 30,3 %-igen wässrigen Formaldehyd-Lösung eingepumpt (Molverhältnis Crotonaldehyd : Acetaldehyd = 1 : 1 ; kalkulatorisches Molverhältnis Acetaldehyd : Formaldehyd = 1 : 0,79). Dabei variiert der Reaktionsdruck zwischen 32 und 33 Bar. Nach beendeter Zugabe des Aldehydgemisches wurde die Reaktionsmasse während 10 Minuten bei 230 °C weitergerührt und dann auf Raumtemperatur abgekühlt. Schließlich erfolgt eine Extraktion mit 3 × 100 ml Methylenchlorid sowie eine gaschromatographische Analyse der vereinigten Methylenchloridextrakte, wobei sich folgende Produkte mit den je nach theoretischem Aldehydbedarf auf eingesetzten Acetaldehyd und Crotonaldehyd (A) oder auf eingesetzten Formaldehyd (F) bezogenen Ausbeuten ergeben : Pyridin 1,0 % (A) ; 3-Picolin 53,5 % (F) ; 3-Ethylpyridin 21,6 % (A) ; 2,5-Lutidin 5,9 % (A) ; 3,5-Lutidin 0,7 % (F) ; 2-Methyl-5-ethylpyridin (A).

Beispiel 9

1 700 ml einer 3,41 molaren wässrigen Lösung von Diammoniumhydrogenphosphat (pH 8,3) werden in einem 2-Liter-Autoklaven auf 230 °C erhitzt und bei 1 500 UpM gerührt. In diese Lösung wird mit einer ersten Pumpe (360,7 g/Std.) ein Gemisch aus 2 214 g Acetaldehyd und 3 985 g einer 30,5 prozentigen wässrigen Formaldehyd-Lösung eindosiert (Molverhältnis 1 : 0,81). Nach Ablauf von einer Stunde erfolgt dann die Zuschaltung einer zweiten Pumpe (1 325,7 g/Std.), mit welcher nun noch 3,41 molare wässrige Lösung von Diammoniumhydrogenphosphat (pH 8,3) zudosiert wird. Das Reaktionsgemisch beginnt jetzt durch ein am Autoklavendeckel angebrachtes Rohr in ein auf gleiche Temperatur erhitztes Auffanggefäß überzulaufen. Nach ca. 15 Minuten werden beide Pumpen gestoppt, die Gewichte der Eduktbehälter registriert, das übergelaufene Reaktionsgemisch in ein Abkühlgefäß abgelassen und dann die Pumpen wieder in Betrieb gesetzt. Dieser Vorgang wird alle 60 Minuten wiederholt, so daß jede Stunde eine Fraktion anfällt. Der in der Zwischenzeit gemessene Reaktionsdruck variiert zwischen 33 und 35 Bar. Nach Beendigung des Versuchs werden die Fraktionen 5-12 aufgearbeitet. Dabei wird jeweils zuerst die organische Phase abgetrennt und die wässrige Phase drei mal mit 100 ml Methylenchlorid extrahiert. Die Extrakte werden mit der oben erwähnten organischen Phase vereinigt und die dabei erneut ausfallende wässrige Phase mit weiteren 60 ml Methylenchlorid ausgeschüttelt. Sämtliche Methylenchlorid-Extrakte der Fraktionen 5-12 werden dann vereinigt und unter Verwendung eines internen Standards sowie von Flächenkorrekturfaktoren gaschromatographisch analysiert. Dabei ergeben sich die folgenden Produkte mit den je nach theoretischem Aldehydbedarf auf Acetaldehyd (A) bw. Formaldehyd (F) bezogenen Ausbeuten : Pyridin 1,2 % (A) ; 3-Picolin 64,1 % (F) ; 3-Ethylpyridin 21,0 % (A) ; 2,5-Lutidin 3,5 % (A) ; 3,5-Lutidin 1,1 % (F) ; 2-Methyl-5-ethylpyridin 1,6 % (A).

## Ansprüche

1. Verfahren zur Herstellung von 3-Picolin, dadurch gekennzeichnet, daß man Acetaldehyd bzw. dessen Polymere und/oder Crotonaldehyd im Gemisch mit Formaldehyd bzw. dessen Polymere in wäßriger Phase bei einem Molverhältnis von Acetaldehyd : Formaldehyd von 1 : 0,5 bis 1 : 1,2 und/oder Crotonaldehyd : Formaldehyd von 1 : 1,0 bis 1 : 2,4 bei Temperaturen von 180 bis 280 °C im geschlossenen Gefäß in Gegenwart von 0,5 bis 3 Mol Ammoniumsalze pro Mol Aldehyd umsetzt.

2. Verfahren gemäß Anspruch 1, dadurch gekennzeichnet, daß man als Ammoniumsalz Ammoniumphosphate anwendet.

3. Verfahren gemäß Ansprüchen 1 und 2, dadurch gekennzeichnet, daß man bei Temperaturen von 205 bis 240 °C arbeitet.

4. Verfahren gemäß Ansprüchen 1 bis 3, dadurch gekennzeichnet, daß man die Ammoniumsalze in wäßriger Lösung in einer Konzentration von 2,9 bis 3,7 Mol/l anwendet.

## Claims

1. Process for the preparation of 3-picoline, characterised in that one reacts acetaldehyde or its polymers and/or crotonaldehyde in admixture with formaldehyde or its polymers in aqueous phase in a mole ratio of acetaldehyde : formaldehyde of 1 : 0.5 to 1 : 1.2 and/or crotonaldehyde : formaldehyde of 1 : 1.0 to 1 : 2.4 at temperatures of 180 to 280 °C in a closed vessel in the presence of 0.5 to 3 mole of ammonium salts per mole of aldehyde.

2. Process according to claim 1, characterised in that one uses ammonium phosphate as ammonium salt.

3. Process according to claims 1 and 2, characterised in that one operates at temperatures of 205 to 240 °C.

4. Process according to claims 1 to 3, characterised in that one uses the ammonium salts in aqueous solution in a concentration of 2.9 to 3.7 mole/l.

## Revendications

1. Procédé de préparation de la 3-picoline, caractérisé en ce que l'on fait réagir l'acétaldéhyde ou ses polymères et/ou l'aldéhyde crotonique en mélange avec du formaldéhyde ou ses polymères en phase aqueuse à un rapport molaire acétaldéhyde/formaldéhyde de 1 : 0,5 à 1 : 1,2 et/ou aldéhyde crotonique/formaldéhyde de 1 : 1,0 à 1 : 2,4 à des températures de 180 à 280 °C dans un récipient fermé en présence de 0,5 à 3 moles de sels d'ammonium par mole d'aldéhyde.

2. Procédé selon la revendication 1, caractérisé en ce que l'on utilise en tant que sel d'ammonium, des phosphates d'ammonium.

3. Procédé selon les revendications 1 et 2, caractérisé en ce que l'on opère à des températures de 205 à 240 °C.

4. Procédé selon les revendications 1 à 3, caractérisé en ce que l'on utilise les sels d'ammonium en solution aqueuse à une concentration de 2,9 à 3,7 moles/litre.